# EUROPEAN PATENT APPLICATION

(11) **EP 4 317 152 A1**
(43) Date of publication of application: **07.02.2024**
(21) Application number: 22775863.8
(22) Date of filing: 25.03.2022
(51) Int. Cl.: C07D 403/12

(54) **COMPOUND, METHOD FOR PRODUCING AND METHOD FOR STORING COMPOUND, METHOD FOR PRODUCING TARGETING AGENT, AND COMPOSITION**

(30) Priority: 26.03.2021 JP 2021053783
(71) Applicant: Nihon Medi-Physics Co., Ltd., Tokyo 136-0075 (JP)
(72) Inventor: WATANABE, Yurie, Tokyo 136-0075 (JP); OSAWA, Sayuri, Tokyo 136-0075 (JP)
(74) Representative: Zabel, Julia Elisabeth
(86) International application number: PCT/JP2022/014681
(87) International publication number: WO 2022/203082

(57) **Abstract**

In this production method, a ligand compound capable of being coordinated with a metal ion and a first compound having an atomic group capable of a click reaction are reacted to obtain a product comprising a second compound having in the structure thereof a ligand and an atomic group capable of a click reaction. Thereafter, an eluting solution which includes a water-soluble organic acid having prescribed physical properties and does not include a trifluoroacetic acid is used to subject the product to liquid chromatography to obtain the second compound which has been purified. The present invention also provides: a method for storing the second compound; a composition including the second compound; and a method for producing a targeting agent using the second compound.

## Description

### Technical Field

The present invention relates to a compound, a manufacturing method and a storing method for a compound, a manufacturing method for a targeting agent, and a composition.

### Background Art

For the purpose of use in reagents and diagnostic agents for detecting a target molecule, or pharmaceuticals for treating diseases, studies are in progress on production conditions and storage conditions for compounds capable of coordinating to various metals such as radioactive metals, for example.

Patent Literature 1 discloses a method in which DOTAGA-DBCO obtained as the above compound is purified and then used to produce a polypeptide labeled with a radioactive metal.

In addition, Non-Patent Literature 1, relating to a method for producing a nanoparticle labeled by coordinating a DOTA derivative to ⁶⁴Cu as a radioactive metal, discloses that trifluoroacetic acid is used for purification in producing DO3A-DBCO, which is the above compound.

### Citation List

### Patent Literature

Patent Literature 1: WO 2019/125982 A1

### Non Patent Literature

Non Patent Literature 1: Zeng et al, ACS Nano. 2012, 6 (6), 5209-5219.

### Summary of Invention

Incidentally, regarding a compound capable of coordinating to a metal ion, it is common in the present technical field to use a solvent or an eluent containing trifluoroacetic acid (Hereinafter, it is also referred to as "TFA" in the present specification.) as disclosed in Non Patent Literature 1 for purifying a ligand compound having a structure containing a carboxy group such as DOTA and a derivative thereof.

However, as shown in Comparative Example 1 described later, it has become clear from the present inventor's findings that, when the compound is purified and stored using a solvent or an eluent containing TFA, the purity of the objective compound may decrease with the lapse of time. From such findings, a technique for enhancing the storage stability of an objective compound has been desired.

Accordingly, an object of the present invention is to enhance the storage stability of an objective compound.

The present invention provides a manufacturing method for a compound, the manufacturing method including: reacting a ligand compound capable of coordinating to a metal ion with a first compound having an atomic group capable of a click reaction to obtain a product that contains a second compound with a structure having an atomic group capable of a click reaction and a ligand; and subsequently,
subjecting the product to a liquid chromatography method using an eluent that contains a water-soluble organic acid that is liquid under 1 atm at 20 °C and contains no trifluoroacetic acid to obtain the purified second compound.

The present invention provides a manufacturing method for a targeting agent, the manufacturing method including in this order or in the reverse order:
a step of coordinating the second compound obtained by the manufacturing method to a metal ion; and
a step of subjecting the second compound and a targeting compound having an atomic group capable of a click reaction to a reaction between atomic groups capable of a click reaction.

The present invention provides a composition including:
a compound with a structure having a ligand capable of coordinating to a metal ion and an atomic group capable of a click reaction;
a water-soluble organic acid that is liquid under 1 atm at 20 °C; and no trifluoroacetic acid.

The present invention provides a compound including a structure having a ligand capable of coordinating to a metal ion and an atomic group capable of a click reaction,
wherein, after the compound is stored at -20 °C for 1 month, the compound has a purity decreasing rate of 5% or less relative to a purity that the compound has when the compound starts to be stored.

The present invention provides a storing method for a compound, the storing method including: freezing and storing the second compound purified by the manufacturing method.

### Description of Embodiments

Hereinafter, the present invention will be described based on preferred embodiments thereof. The manufacturing method of the present invention has: a step of reacting a ligand compound capable of coordinating to a metal ion with a first compound having an atomic group capable of a click reaction to obtain a product that contains a second compound with a structure having an atomic group capable of a click reaction and a ligand (Hereinafter, the step is also referred to as "synthesizing step".); and a step of purifying the product obtained through the synthesizing step under predetermined conditions to obtain the purified second compound (Hereinafter, the step is also referred to as "purifying step".).

In the synthesizing step, a ligand compound and a first compound are reacted to obtain a product containing a second compound. From the viewpoint of uniformity in the reaction, the reaction of both compounds is preferably performed in a state where both compounds are dissolved or dispersed in a reaction solvent.

Details of the ligand compound, the first compound, and the second compound, as well as reaction conditions thereof, will be described later.

The product obtained through the synthesizing step, due to a reaction of the ligand compound and the first compound, contains an unreacted ligand compound and first compound, by-products other than the second compound, such as a regioisomer, and a reaction solvent as needed, in addition to the second compound as an objective main product.

The second compound is an organic compound having two chemical structures: a ligand capable of coordinating to a metal ion derived from the chemical structure of the ligand compound; and an atomic group capable of a click reaction derived from the chemical structure of the first compound. That is, the second compound has a structure having a chemical structure capable of coordinating to a metal ion and a chemical structure capable of a click reaction.

The present manufacturing method employs predetermined conditions in the purifying step for purifying the product containing the above second compound, as one of its characteristics.

Conventionally, a solvent or an eluent containing TFA has been used to purify an organic compound with a structure having a ligand. However, it has become clear that such conditions may result in failing to appropriately separate and purify the objective compound or decreasing the purity of the objective compound with the lapse of time in storing the purified compound. As a result of intensive studies on why it is by the present inventor, it has been presumed that TFA is unintentionally coordinated or bonded to a ligand or unintentionally reacts with a highly reactive functional group.

From such findings, the present inventor has found that: in the purifying step, purification under the condition where an organic acid other than TFA is used and TFA is not contained unexpectedly makes it possible to achieve purification in high purity, suppress the decomposition of the objective compound obtained by the purification, and improve storage stability.

In the purifying step, the product containing the second compound is subjected to a liquid chromatography method. The eluent used at this time is preferably one that contains a water-soluble organic acid that is liquid under 1 atm at normal temperature and does not contain TFA. The water-soluble organic acid is more preferably one that is liquid under 1 atm at normal temperature and has a boiling point of 150 °C or less under 1 atm. By employing such conditions, the eluent can be transpired without being left when a liquid composition containing the purified second compound is dried under reduced pressure or freeze-dried to obtain the solid second compound. As a result, purification efficiency can be enhanced and storage stability can be improved.

The "water-soluble organic acid" in the present specification means an organic acid that is dissolved in water at 20°C under 1 atm. The water-soluble organic acid preferably has a physical property that, when the organic acid and water are mixed, layer separation is not visually observed and a uniform state is obtained. More preferably, the water-soluble organic acid in the present specification means an organic acid that is dissolved in 3 mL or more with respect to 100 mL of water. It is more preferable that the water-soluble organic acid has a physical property that when the organic acid and water are mixed at a volume ratio of 1:10 (organic acid : water), layer separation is not visually observed and a uniform state is obtained.

In addition, in the present specification, "not containing TFA" means that TFA is not intentionally contained in the reaction system or in the obtained compound or composition. It is allowed that TFA present in a trace amount in raw materials or TFA remaining in a trace amount in measuring instruments are inevitably mixed. Taking the eluent as an example, TFA is not intentionally contained in the eluent, but for example, it is allowed that TFA derived from raw materials is inevitably mixed in the eluent.

The absence of TFA can be determined, for example, when ¹⁹F-NMR analysis observes no peak derived from TFA.

Examples of the liquid chromatography method used in the purifying step include at least one of column chromatography using a column packed with various fillers or a gel, high performance liquid chromatography, intermediate pressure preparative liquid chromatography, and the like.

The water-soluble organic acid suitably used in the purifying step preferably has a boiling point of 150°C or lower under 1 atm, and specific examples thereof include formic acid (boiling point: 100.8°C), acetic acid (boiling point: 118°C), and propionic acid (boiling point: 141.2°C). These can be used alone or in combination.

Among them, it is preferable to use acetic acid from the viewpoint of achieving all of convenience in handling, improvement in purification efficiency, suppression of unintended chemical reaction with the second compound, and improvement in storage stability of the purified second compound.

The concentration of the water-soluble organic acid in the eluent is preferably 0.001 vol% or more and 10 vol% or less, more preferably 0.01 vol% or more and 1.0 vol% or less, and still more preferably 0.1 vol% or more and 1.0 vol% or less. When the concentration is in such a range, purification efficiency can be further improved.

Examples of the liquid component other than the water-soluble organic acid constituting the eluent include: water, such as distilled water and ion-exchanged water; water-soluble protic solvents, such as methanol and ethanol; water-soluble aprotic solvents, such as acetonitrile, N,N-dimethylformamide (DMF), tetrahydrofuran, dimethylsulfoxide, and acetone; and water-insoluble organic solvents, such as hexane, toluene, and ethyl acetate. These can be used alone or in combination. In the present specification, the water-soluble protic solvent and the water-soluble aprotic solvent may be referred to as "polar organic solvent".

Among them, from the viewpoint of dissolving all the ligand compound, the first compound, and the second compound to facilitate purification, the liquid component of the eluent preferably contains at least one of water and a polar organic solvent, and more preferably contains at least one of water and acetonitrile.

From the viewpoint of shortening the purification time, improving purification efficiency, and improving handleability, it is preferable to perform the purifying step using a plurality of kinds of eluent. Specifically, when water and a polar organic solvent are selected as the liquid components of the eluent, exemplified is a method of using a first eluent that is an aqueous solution of the above-described water-soluble organic acid and does not contain TFA, and subsequently using a second eluent that is a solution of the polar organic solvent and contains the above-described water-soluble organic acid and does not contain TFA. Switching between the first eluent and the second eluent may be performed as separate and independent steps, or may be performed within one step while changing these concentration gradients stepwise or continuously (so-called gradient).

The purified second compound can be obtained through the above steps. The second compound is preferably obtained in the form of a composition that is a solution or dispersion in the above-described eluent. That is, the composition contains the second compound with a structure having a ligand capable of coordinating to a metal ion and an atomic group capable of a click reaction, contains a water-soluble organic acid that is liquid under 1 atm at 20 °C, and does not contain TFA.

The purified second compound can be subjected to subsequent steps in the form of a liquid composition, or stored in the form of a composition. Alternatively, the composition can be subjected to a drying method such as vacuum drying or freeze-drying (freezing and drying) to remove the eluent, thereby obtaining the second compound in a solid state. When drying is performed, the second compound may be obtained as a simple substance (a single substance, in other words, a pure substance), or may be in the form of a solid composition in which a part of the organic acid is left.

The solid second compound can be dissolved in another solvent to be subjected to subsequent steps, or stored in a solid state. The storage conditions can be under normal temperature, refrigerating, or freezing. Each of "normal temperature", "refrigerating", and "freezing" used in the present specification are not limited as long as it is a temperature within a general numerical range the term refers to. For example, "normal temperature" may be 15 to 25°C, "refrigerating" may be 0 to 10°C, and "freezing" may be -100 to 0°C.

Among them, from the viewpoint of suppressing the decomposition of the second compound by the eluent and suppressing an increase in impurities, it is preferable to freeze-dry the purified second compound to obtain the solid second compound.

In addition, regardless of whether or not the second compound is subjected to drying, it is also preferable to freeze and store the purified second compound from the viewpoint of suppressing the decomposition of the second compound and an increase in impurities.

The temperature in the freeze-drying is set to preferably lower than 0°C, more preferably -15°C or lower, and still more preferably -70°C or lower in terms of the product temperature of the objective compound. The external pressure in freeze-drying may be normal pressure or negative pressure.

In addition, the second compound purified through each of the above-described steps has a purity decreasing rate of preferably 5% or less, more preferably 3% or less, still more preferably 2% or less relative to a purity that the second compound has when the second compound starts to be stored, after the second compound is stored at -20 °C or less for 1 month.

The purity of the compound at the start of storage and after storage each can be calculated through area percentage method after calculating the peak area with automatic integration method using a result measured by high performance liquid chromatography.

Hereinafter, what is commonly applicable to each of the above-described embodiments will be described.

The ligand compound is not particularly limited as long as it is an organic compound capable of coordinating to a metal ion, and examples thereof include the following organic compounds and compounds containing a structure derived from the compounds.

<CDTA or derivative thereof>
   CB-TE2A (1,4,8,11-Tetraazabicyclo[6.6.2]hexadecane-4,11-diacetic acid)
   CDTA (Cyclohexane-trans-1,2-diamine tetra-acetic acid)
   CDTPA (4-cyano-4-[[(dodecylthio)thioxomethyl]thio]-Pentanoic acid)
<DOTA or derivative thereof>
   DOTA (1,4,7,10-Tetraazacyclododecane-1,4,7,10-tetraacetic acid)
   DOTMA ((1R,4R,7R,10R)-α,α',α",α‴-tetramethyl-1,4,7,10-tetraazacyclododecane-1,4,7,10-tetraacetic acid)
   DOTAM (1,4,7,10-tetrakis(carbamoylmethyl)-1,4,7,10-tetraazacyclododecane)
   DOTAGA (α-(2-Carboxyethyl)-1,4,7,10-tetraazacyclododecane-1,4,7,10-tetraacetic acid)
   DOTP (((1,4,7,10-Tetraazacyclododecane-1,4,7,10-tetrayl)tetrakis(methylene))tetraphosphonicacid)
   DOTMP (1,4,7,10-Tetraazacyclododecane-1,4,7,10-tetrakis(methylenephosphonic acid))
   DOTA-4AMP (1,4,7,10-tetraazacyclododecane-1,4,7,10-tetrakis(acetamidomethylenephosphonic acid)
   DO2P (Tetraazacyclododecane dimethanephosphonic acid)
   p-SCN-Bn-DOTA(2-[(4-isothiocyanatophenyl)methyl]-1,4,7,10-Tetraazacyclododecane-1,4,7,10-tetraacetic acid)
<DTPA or derivative thereof>
   DTPA(N,N-bis[2-[bis(carboxymethyl)amino]ethyl]-glycine)
   DTPA-BMA(5, 8-Bis(carboxymethyl)-11-[2-(methylamino)-2-oxoethyl]-3-oxo-2,5,8,11-tetraazatridecan-13-oic acid)
<TETA, HEHA, PEPA, or derivative thereof>
   TETPA (1,4,8,11 -tetraazacyclotetradecane-1,4,8,11-tetrapropionic acid)
   TETA (1,4,8,11-Tetraazacyclotetradecane-N,N',N",N‴-tetraacetic acid)
   TTHA(3,6,9,12-Tetrakis(carboxymethyl)-3,6,9,12-tetraazatetradecanedioic acid)
   HEHA (1,2,7,10,13-hexaazacyclooctadecane-1,4,7,10,13,16-hexaacetic acid)
   1,2-HOPO (N,N',N",N‴-tetra(1,2-dihydro-1-hydroxy-2-oxopyridine-6-carbonyl)-1,5,10,14-tetraazatetradecane)
   PEPA (1,4,7,10,13-pentaazacyclopentadecane-N,N',N",N‴,Nʺʺ-penta-acetic acid)
   OTP (1,4,7-Triazacyclononane-1,4,7-triyltris(methylenephosphonic acid)
<NOTA>
   NOTA (1,4,7-Triazacyclononane-1,4,7-triacetic acid)
<Chain ligand compound>
   Deferoxamine(DFO)
   EDTA (2,2',2",2‴-(ethane-1,2-diylbis(azanetriyl))tetraacetic acid)
<Macropa or derivative thereof>
   H2macropa (6-(1,4,10,13-tetraoxa-7,16-diazacyclooctadecan-N,N'-methyl)picolinic acid)
   macropa-NH₂ (4-amino-6-[[16-[(6-carboxypyridin-2-yl)methyl]-1,4],10,13-tetraoxa-7,16-diazacyclooctadec-7-yl]methyl)pyridine-2-carboxylic acid)
   macropa-NCS (6-[[16-[(6-carboxypyridin-2-yl)methyl]-1,4,10,13-t]]etraoxa-7,16-diazacyclooctadec-7-yl)methyl]-4-isothiocyanatopyridine-2-carboxylic acid)
<Other ligand compounds>
   H4octapa (N,N'-bis(6-carboxy-2-pyridylmethyl)-ethylenediamine-N,N'-diacetic acid)
   H2bispa2 (6,6'-({9-hydroxy-l,5-bis(methoxycarbonyl)-2,4-di(pyridine-2-yl)-3,7-diazabicyclo[3.3.1]nonane-3,7-diyl}bis(-methylene))dipicolinic acid)
   H2dedpa (1,2-[{6-(carboxy)-pyridin-2-yl}-methylamino]ethane)
   H5decapa (N,N"-bis(6-carboxy-2-pyridylmethyl)-diethylenetriamine-N,N',N"-triacetic acid)
   H6phospa (N,N'-(methylenephosphonate)-N,N'-[6-(methoxycarbonyl)pyridin-2-yl]-methyl-1,2-diaminoethane)
   HP-DO3A(Hydroxypropyltetraazacyclododecanetriaceticacid)
   HBED-CC (N,N'-Bis[2-hydroxy-5-(carboxyethyl)-benzyl]ethylenediamine-N,N'-diacetic acid)

Among them, from the viewpoint of achieving both convenience in subsequent production steps and improvement in complexing properties, the ligand compound used in the synthesizing step is preferably Macropa, Deferoxamine, HBED-CC, CDTA, DOTA, DTPA, NOTA, or a derivative thereof, and more preferably a compound having a structure derived from DOTA or a derivative thereof. Examples of the structure derived from DOTA include those represented by the following formula (1). These compounds may be an anhydride, a hydrate, or an acid anhydride.

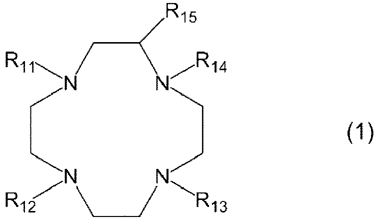

In the formula (1), R₁₁, R₁₂, and R₁₃ each independently represent a group consisting of -(CH₂)ₚCOOH, -(CH₂)ₚC₅H₄N, -(CH₂)ₚPO₃H₂, or -(CH₂)ₚCONH_{2.}

The "p" is each independently an integer of 0 or more and 3 or less.

In the formula (1), one of R₁₄ and R₁₅ is a hydrogen atom, or a group consisting of -(CH₂)_{pCOOH}, -(CH₂)ₚC₅H₄N, -(CH₂)ₚPO₃H₂, -(CH₂)ₚCONH₂, or - (CHCOOH)(CH₂)ₚCOOH.

In the formula (1), the other of R₁₄ and R₁₅ is a group consisting of -(CH₂)_{pCOOH}, -(CH₂)ₚC₅H₄N, -(CH₂)ₚPO₃H₂, or -(CH₂)ₚCONH₂, or an atomic group to link the first compound described later.

The "p" is each independently an integer of 0 or more and 3 or less.

The first compound used in the synthesizing step has a structure having an atomic group capable of a click reaction. Examples of such an atomic group include: an alkynyl group or an azido group; or a diene or a dienophile, such as 1,2,4,5-tetrazine or an alkenyl group. These are also preferably an atomic group that can be used for metal catalyst-free click reactions.

The click reaction is a reaction caused by a combination of an alkyne and an azide, or a combination of a diene and a dienophile, such as 1,2,4,5-tetrazine and an alkene. Specific examples of a click reaction by such a combination of atomic groups include Huisgen cycloaddition reaction and inverse electron demand Diels-Alder reaction.

Typically, the chemical structure produced by a click reaction in a combination of an alkyne and an azide contains a triazole skeleton, and the chemical structure produced by a click reaction in a combination of 1,2,4,5-tetrazine and an alkene as a combination of a diene and a dienophile contains a pyridazine skeleton.

Specific examples of the atomic group capable of a click reaction include, as shown in the following formulas, an atomic group containing dibenzocyclooctyne (DBCO) as an alkyne (formula (5a)), an atomic group containing an azido group as an azide (formula (5b)), an atomic group containing 1,2,4,5-tetrazine (formula (5c)), and an atomic group containing trans-cyclooctene (TCO) as an alkene (formula (5d)).

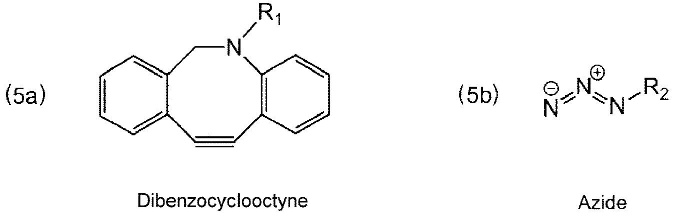

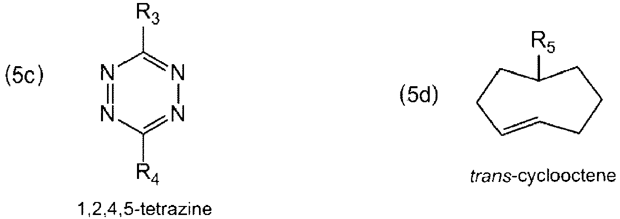

In the formula (5a), R₁ represents a bonding part with another structure.

In the formula (5b), R₂ represents a bonding part with another structure.

In the formula (5c), one of R₃ and R₄ represents a bonding part with another structure, and the other represents a hydrogen atom, a methyl group, a phenyl group, or a pyridyl group.

In the formula (5d), R₅ represents a bonding part with another structure.

From the viewpoint of easily introducing an atomic group capable of a click reaction into the objective second compound and from the viewpoint of simplicity in the reaction conditions of the second compound, the atomic group capable of a click reaction in the first compound is preferably dibenzocyclooctyne (DBCO).

As the first compound, various commercially available reagents may be used. Specifically, when introducing an atomic group containing DBCO as the atomic group capable of a click reaction, for example, DBCO reagents, such as DBCO-C6-acid, DBCO-amine, DBCO-maleimide, DBCO-PEGacid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, and DBCO-mPEG, can be used.

The reaction between the ligand compound and the first compound in the synthesizing step is preferably performed in a state where both the compounds are dissolved or dispersed in a reaction solvent. Examples thereof include: a method in which the solid ligand compound and the solid first compound are dissolved or dispersed in a reaction solvent; and an aspect in which a solution or dispersion of one compound is added with the other compound in a solid or liquid form. If necessary, the reaction system may be added with another compound that is a compound other than the ligand compound and the first compound and can form a linker structure. Examples of such another compound include amino acids, polyethylene glycol (PEG), and the like.

As the reaction solvent used in the synthesizing step, for example, the same solvent as the solvent described in the above eluent can be used alone or in combination.

When the reaction is performed in a reaction liquid in the synthesizing step, the concentration of the ligand compound in the reaction liquid can be appropriately changed depending on the type of compound, and is preferably 0.12 mol/L or more and 0.44 mol/L or less, and more preferably 0.26 mol/L or more and 0.31 mol/L or less from the viewpoint of improving reaction yield.

From the same viewpoint, the concentration of the first compound in the reaction liquid can be appropriately changed depending on the type of compound, and the lower limit of the concentration of the first compound in the reaction liquid is preferably 0.10 mol/L or more and more preferably 0.20 mol/L or more. The upper limit of the concentration of the first compound in the reaction liquid is preferably 0.30 mol/L or less and more preferably 0.24 mol/L or less. From the viewpoint of improving reaction yield, preferable is 0.10 mol/L or more and 0.22 mol/L or less and more preferable is 0.22 mol/L or more and 0.24 mol/L or less.

From the same viewpoint, the molar ratio of the ligand compound to the first compound is preferably 1.2 or more and 3.0 or less, more preferably 1.2 or more and 2.0 or less, and particularly preferably 1.2 or more and 1.3 or less.

In the synthesizing step, from the viewpoint of achieving further improvement in yield in a short reaction time, it is preferable to perform the reaction by heating the reaction liquid. The heating means applying heat from outside the reaction system such that, on the basis of 25°C, the temperature of the reaction liquid is higher than 25°C. As a method for applying heat from outside the reaction system, a known method can be appropriately used, and examples thereof include a water bath, an oil bath, a block heater, and a mantle heater.

When the reaction liquid is heated for the reaction, the reaction liquid is heated to a reaction temperature of preferably 30°C or more and 100°C or less, more preferably 50°C or more and 80°C or less, from the viewpoint of achieving both suppression of the decomposition of the ligand compound and further improvement in yield.

The reaction time is preferably 2 hours or more and 24 hours or less, and more preferably 2 hours or more and 4 hours or less on condition that the reaction temperature is as described above.

The reaction pressure can be atmospheric pressure.

In the reaction between the ligand compound and the first compound, it is preferable to select conditions that the ligand compound's chemical structure capable of coordinating to a metal ion and the first compound's chemical structure of the atomic group capable of a click reaction are not particularly modified and both the chemical structures are maintained from the viewpoint of enhancing the convenience of the subsequent production processes.

In addition, regarding the bonding mode between the ligand compound and the first compound, it is preferable to select conditions that a stable bond is formed under normal synthesis conditions, from the viewpoint that the decomposition of the obtained compound can be suppressed and the storage stability of the obtained compound can be further improved.

Preferable examples of the reaction method capable of achieving both of the above-described suitable conditions preferably include a method in which the ligand compound and the first compound are subjected to an amidation reaction to bond both of the compounds through an amide bond. It is also preferable that these reactions are carried out at the side chain of each compound.

Specific examples thereof include a method of using a ligand compound with a structure containing a carboxy group and a first compound with a structure containing an amino group and performing an amidation reaction between the carboxy group and the amino group to obtain a second compound with a structure having an amide bond. The carboxy group is a monovalent functional group represented by "-COOH" or "-COO⁻". The amino group is a monovalent functional group represented by "-NH₂" or "-NH₃⁺".

The amidation reaction can also be carried out using the ligand compound or the first compound having an oxydicarbonyl group instead of a carboxy group. The oxydicarbonyl group is a divalent functional group represented by "-C(=O)OC(=O)-", and a compound having the functional group is referred to as an acid anhydride or simply an anhydride. The ligand compound or the first compound having an oxydicarbonyl group may be a symmetrical anhydride having two identical acyl groups or a mixed anhydride having different acyl groups. A cyclic anhydride produced by a dehydration condensation between carboxy groups present in the same molecule of a polycarboxylic acid may be used. Among them, from the viewpoint of economic efficiency and synthesis efficiency, a mixed anhydride or a cyclic anhydride is preferable, and a cyclic anhydride is more preferable.

As the reaction conditions, the amidation reaction may be carried out by stirring in a reaction solvent at room temperature or under heating, and may be carried out by adding an amide condensing agent as necessary.

Examples of the amide condensing agent to be used include: carbodiimide condensing agents, such as N,N '-dicyclohexylcarbodiimide (DCC); condensing agents via an acid azide, such as diphenylphosphate azide (DPPA); BOP reagent combining hexamethylphosphoric acid triamide (HMPA) and 1-hydroxybenzotriazole (HOBt); PyBOP, in which the dimethylamino group of BOP reagent is substituted with a pyrrolidino group; uronium type condensing agents, such as O-(benzotriazole-1-yl)-N,N,N',N'-tetramethyluronium hexafluorophosphate (HBTU), in which the phosphorus of the BOP reagent is substituted with carbon; and triazole type condensing agents, such as DMT-MM.

In addition, the amidation reaction may be performed by adding a base, such as triethylamine, as necessary.

Another preferable examples of the reaction method capable of achieving both of the above-described suitable conditions preferably include a method in which a reaction to form a thiourea structure between the ligand compound and the first compound is performed to bond both the compounds. It is also preferable that these reactions are carried out at the side chain of each compound.

Specific examples thereof include a method of using the ligand compound with a structure containing an isothiocyanate group and the first compound with a structure containing an amino group and performing a reaction between the isothiocyanate group and the amino group to obtain the second compound with a structure having a thiourea structure. The isothiocyanate group is a functional group represented by "-N=C=S". The thiourea structure is a chemical structure represented by "-NH-C(=S)-NH-".

The reaction conditions for forming a thiourea structure can be, for example, conditions that a base such as N,N'-diisopropylethylamine or triethylamine is used and stirring is performed at room temperature or under heating.

Examples of the second compound with a structure having an amide bond include, but are not limited to, compounds represented by the following formulas (7a) and (7b).

Examples of the second compound with a structure having a thiourea structure include, but are not limited to, compounds represented by the following formulas (8a) and (8b).

All of the second compounds represented by the following formulas (7a) and (7b) and formulas (8a) and (8b) maintain both the chemical structure capable of coordinating to a metal ion and the first compound's chemical structure of an atomic group capable of a click reaction.

The chemical structure of the second compound can be appropriately changed depending on the type of the ligand compound and the first compound to be used.

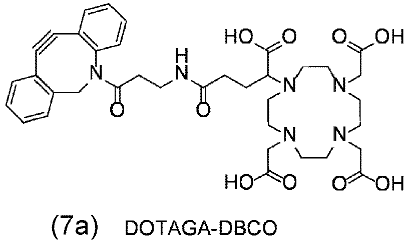

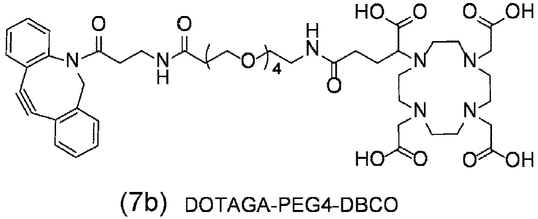

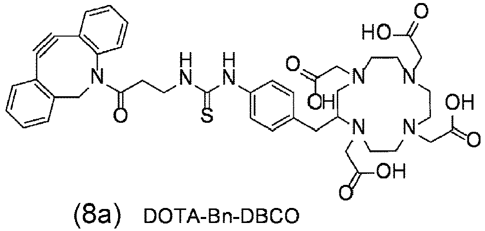

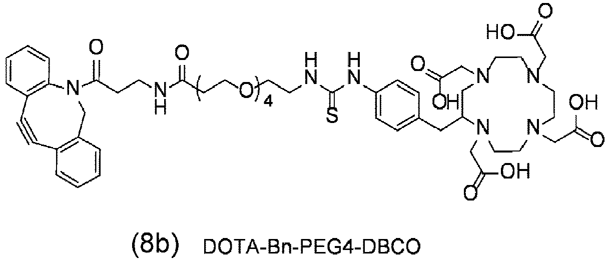

From the viewpoint of obtaining the second compound having higher storage stability, and from the viewpoint of more efficiently performing a coordination reaction of a metal ion and a click reaction in the subsequent steps, it is preferable that the ligand compound is DOTAGA or an anhydride thereof, and the first compound is DBCO-amine. The second compound obtained by reacting these compounds is DOTAGA-DBCO, which is represented by the above formula (7a). An embodiment of the reaction route is shown below.

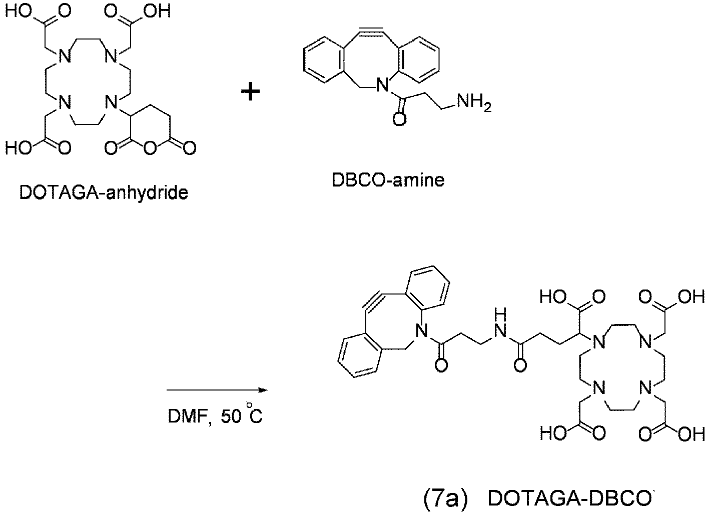

The second compound obtained through the purifying step can be subjected to the subsequent steps in a state of a simple substance or a composition, in a state of having been dissolved in a solvent, a buffer solution or the like, or in a state of having been subjected to a step of distilling off the predetermined eluent under reduced pressure or the like.

Examples of the step of using the second compound obtained through the purifying step include the following steps (a) and (b). These steps are an embodiment of the method for manufacturing a targeting agent. The targeting agent has a chemical structure including: a metal complex; and an atomic group having directivity to a target organ or tissue in a living body or a specific bonding ability to a target molecule.

Step (a): a step of coordinating the second compound (specifically, the ligand contained in the chemical structure of the second compound) to a metal ion to form a metal complex (complex-forming step).

Step (b): a step of subjecting the second compound and a targeting compound having an atomic group capable of a click reaction to a reaction between atomic groups capable of a click reaction (click reaction step).

The steps (a) and (b) described above may be performed in the order of (a) and (b), or may be performed in the order of (b) and (a) instead. In addition, the steps (a) and (b) may be continuously performed in this order or in the reverse order. Alternatively, another step other than the steps (a) and (b) may be interposed between the step (a) and the step (b).

In the manufacturing method for a targeting agent, it is preferable to perform steps (a) and (b) in this order. Specifically, it is preferable to perform the step of coordinating the second compound obtained through the purifying step to a metal ion to obtain a metal complex, and subsequently perform the step of subj ecting the metal complex and a targeting compound having an atomic group capable of a click reaction to a reaction between atomic groups capable of a click reaction. It is advantageous to perform each of the steps in this order in that the yield of the complex can be increased, and the targeting agent can be subjected to subsequent steps without separating and purifying unreacted metal ions. In addition, a targeting agent having an enhanced directivity to a target organ or tissue in a living body or specific bonding ability to a target molecule can be obtained in high productivity. In particular, it is advantageous to use a radioactive metal complex in that treatment and detection of a disease can be effectively performed.

Hereinafter, a preferable manufacturing method for the targeting agent will be described referring to a case where the steps (a) and (b) are performed in this order, as an example.

The second compound obtained through the purifying step can be reacted with a metal ion in a state of a simple substance or a composition or in a state of having been dissolved in a solvent, or in a solution such as a buffer solution to form a metal complex (complex-forming step).

The metal complex obtained in this step is formed by coordinating a metal ion to the ligand included in the chemical structure of the second compound. The metal to be coordinated may be a non-radioactive element or a radioisotope.

From the viewpoint of enhancing complex formation efficiency, the metal to be reacted with the second compound is preferably used in the form of an ionizable metal compound, and more preferably used in the form of a metal ion (Hereinafter, these forms are also collectively referred to as "metal source".). As the metal source, for example, a metal ion-containing liquid in which the metal is dissolved in the state of a metal ion in a solvent mainly composed of water can be used.

In addition, from the viewpoint of enhancing complex formation efficiency regardless of the combination of the ligand and the metal in the structure of the second compound, it is preferable to heat and react the reaction system containing the compound and the metal ion in complex formation.

The heating condition is preferably 30°C or higher and 100°C or lower, and more preferably 50°C or higher and 80°C or lower.

The heating time can be appropriately changed depending on the type of the metal to be used. On condition that the above-described temperature range is employed, the lower limit is preferably 5 minutes or more, more preferably 10 minutes or more, even more preferably 15 minutes or more, and still more preferably 30 minutes or more, and the upper limit is preferably 150 minutes or less, more preferably 120 minutes or less,even more preferably 100 minutes or less, and still more preferably 90 minutes or less.

The amount of the reaction liquid is not particularly limited. From the viewpoint of practicality, 0.01 mL to 100 mL is practical at the start of this step. In addition, the concentrations of the compound and the metal ion in the reaction liquid are each independently preferably 1 µmol/L to 100 µmol/L at the start of this step from the viewpoint of the yield of the objective metal complex.

As the solvent used in the complex-forming step, for example, water, saline, or a buffer, such as a sodium acetate buffer, an ammonium acetate buffer, a phosphate buffer, a phosphate buffer saline, a Tris buffer, a HEPES buffer, and a tetramethylammonium acetate buffer, can be used.

Examples of the metal to be coordinated include non-radioactive elements and radioactive elements of alkali metals, alkaline earth metals, lanthanoids, actinoids, transition metals, or metals other than these metals, and isotopes thereof.

Examples of such a metal element include Sc, Cr, Co, Fe, Ga, Cu, Sr, Zr, Y, Tc, Ru, In, Sm, Dy, Ho, Lu, Re, Au, Tl, Hg, Bi, Pb, Th, and Ac. From the viewpoint that the metal complex or the composition containing the same can be applied to treatment, diagnosis, or detection of a disease, Zr, Lu, In, Y, Ga, Cu, or Ac is preferable, and ⁸⁹Zr, ¹⁷⁷Lu, ¹¹¹In, ⁹⁰Y, ⁶⁷Ga, ⁶⁸Ga, ⁶⁴Cu, or ²²⁵Ac, which is a radioactive metal, is more preferable.

These metals are produced by a conventional method, and can be obtained as a solution containing the metals in an ionized state.

The metal complex obtained through the complex-forming step has an atomic group capable of a click reaction derived from the chemical structure of the second compound. Therefore, the metal complex is subjected to a click reaction with a targeting compound having a second atomic group capable of a click reaction to produce a targeting agent (click reaction step). As a result, an agent having an enhanced directivity to a target organ or tissue in a living body or specific bonding ability to a target molecule can be obtained. In particular, it is advantageous to use a radioactive metal complex in that treatment and detection of a disease can be effectively performed.

In producing the targeting agent, it is preferable to perform a click reaction between the atomic group capable of a click reaction contained in the chemical structure of the metal complex and the second atomic group capable of a click reaction contained in the chemical structure of the targeting compound.

In an embodiment, when DOTAGA-DBCO is used as the second compound, the metal complex obtained using the second compound has DBCO as the atomic group capable of a click reaction. Thus, a click reaction can be carried out between DBCO and an azido group by using the targeting compound having an azido group as the second atomic group capable of a click reaction.

As the reaction conditions of the click reaction, known conditions can be employed. From the viewpoint of preventing denaturation of the targeting compound, the click reaction is preferably performed in a non-heated state.

The targeting compound preferably contains one kind or two or more kinds of atomic groups selected from chain peptides, cyclic peptides, a combination thereof, proteins, antibodies or fragments thereof, peptide aptamers, growth factors, affibodies, unibodies, nanobodies, monosaccharides, polysaccharides, vitamins, antisense nucleic acids, siRNAs, miRNAs, nucleic acid aptamers, decoy nucleic acids, cPG oligonucleic acids, peptide nucleic acids, liposomes, micelles, carbon nanotubes, and nanoparticles. It is also preferable that these atomic groups have a chemical structure capable of bonding to an objective target molecule.

In addition, in the case of introducing the second atomic group capable of a click reaction into the targeting compound, a known reagent can be used.

These targeting compounds may be directly bonded to the metal complex or may be indirectly bonded through another known linker structure, such as PEG. When the linker structure includes a structure derived from PEG, an indirect bond through a linker structure represented by the following formula (P) is also preferable. In the formula (P), "n" is preferably an integer of 2 or more and 10 or less, more preferably an integer of 2 or more and 8 or less, and still more preferably an integer of 2 or more and 5 or less.

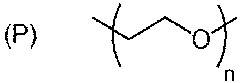

When the targeting compound has a structure including a peptide, the atomic group preferably includes a chain peptide, a cyclic peptide, a combination thereof, a protein, and an antibody or a fragment thereof, each of which specifically bonds to a specific molecule.

Examples of such an atomic group are simply required to be a peptide having three or more constituent amino acid residues, and include: antibodies (immunoglobulins) having a class of IgG, IgA, IgM, IgD, and IgE; antibody fragments, such as Fab fragments and F(ab')2 fragments; and peptide aptamers. The amino acid constituting such a targeting agent may be a natural one or a synthetic one.

The molecular weight of the above atomic group including a peptide is not particularly limited.

The various peptides described above can be synthesized by conventionally known methods, for example, techniques such as liquid phase synthesis method, solid phase synthesis method, automatic peptide synthesis method, genetic recombination method, phage display method, genetic code reprogramming, and RaPID (Random non-standard Peptide Integrated Discovery) method. In the synthesis of various peptides, functional groups of amino acids to be used may be protected as necessary.

When the targeting compound is an atomic group with a structure containing a nucleic acid, the atomic group is preferably an atomic group containing an antisense nucleic acid, siRNA, miRNA, nucleic acid aptamer, decoy nucleic acid, cPG oligonucleic acid, or peptide nucleic acid, each of which specifically bonds to a specific molecule. In addition, the nucleobase may be a natural one such as deoxyribonucleic acid or ribonucleic acid, or may be a synthetic one.

The atomic group containing the above-described nucleic acid that can be used in the present invention can be produced by a conventionally known method. For example, in the case of the nucleic acid aptamer, a nucleic acid aptamer that specifically bonds to a specific target substance, such as a protein, can be produced using the SELEX method (Systematic Evolution of Ligands by Exponential Enrichment).

The metal complex and the targeting agent produced through the above steps are typically present in a state of being dissolved in a reaction liquid. These solutions may be each independently used as they are, or may be purified using a filtration filter, a membrane filter, a column packed with various fillers, chromatography, or the like.

As a step after the targeting agent is obtained, for example, a pharmaceutical preparation step for obtaining a drug containing the targeting agent as an active ingredient can be further performed. In the pharmaceutical preparation step, various additives are appropriately added, such as: a pH adjuster, such as a citrate buffer, a phosphate buffer, and a borate buffer; a solubilizing agent, such as polysorbate; a stabilizer; and an antioxidant, and the concentration of the agent is adjusted by diluting with an isotonic solution such as water and saline. In addition, after adding various additives or adjusting the concentration as the pharmaceutical preparation step, it is possible to include a step of performing sterile filtration with a membrane filter or the like to prepare an injectable preparation.

The ligand compound, the first compound, and the second compound in the present specification may each independently be a compound labeled or isotopically substituted with an isotope of the same element as an element in the structure, and one or more atoms may be substituted with an atom having an atomic mass or mass number different from a naturally occurring atomic mass or mass number.

Examples of the above-mentioned isotopes include isotopes of hydrogen, carbon, nitrogen, and oxygen, such as ²H, ³H, ¹¹C, ¹³C, ¹⁴C, ¹⁵N, ¹⁷O, and ¹⁸O. Pharmaceutically acceptable salts of each of the compounds having the isotopes and/or other isotopes of other atoms are within the scope of the present invention. Compounds incorporated with radioisotopes such as ³H and ¹⁴C are useful in studies for non-clinical or non-medical purposes, such as drug and/or substrate tissue distribution assays. In this case, when a metal complex is formed, the metal ion to be coordinated may be a non-radioactive element. A compound with a structure having at least one of ³H and ¹⁴C is preferable from the viewpoint that the compound is easy to prepare and detect. Isotopically labeled compounds described above can typically be prepared by using an isotopically labeled reagent instead of a non-isotopically labeled reagent.

### Examples

Hereinafter, the present invention will be described in more detail with reference to examples. However, the scope of the present invention is not limited to such examples. The following examples were all carried out under atmospheric pressure.

In the following table, a column indicated with "-" means "not performed".

### [Examples 1 to 4]

### (Synthesizing Step)

An anhydride of DOTAGA as the ligand compound and DBCO-amine as the first compound were used to be dissolved in DMF to obtain a reaction solution. The molar ratio of the ligand compound to the first compound was 3. The reaction solution was heated at 50°C for 3 hours to perform an amidation reaction between the ligand compound's carboxy group and the first compound's amino group, thereby obtaining a product containing DOTAGA-DBCO as the second compound.

### (Purifying Step)

The product containing DOTAGA-DBCO was then subjected to a preparative medium pressure liquid chromatography method using an eluent for purification.

Regarding the eluent in the preparative medium pressure liquid chromatography, a 0.1 vol% acetic acid aqueous solution as the first eluent and a 0.1 vol% acetic acid-containing acetonitrile as the second eluent were used to perform elution under the following concentration gradient conditions. Each of the eluents did not contain TFA.

### <Conditions for preparative medium pressure liquid chromatography >

The first eluent (Hereinafter, it is also referred to as eluent 1.) and the second eluent (Hereinafter, it is also referred to as eluent 2.) were used for liquid feeding while controlling the concentration gradient (vol%) in the order of the following (i) to (iii). The flow rate of each eluent was 12 mL/min.
(i) 0 to 6 min Eluent 1: 80, Eluent 2: 20
(ii) 6 to 34 min Eluent 1: 80 to 58, Eluent 2: 20 to 42
(iii) 34 to 40 min Eluent 1: 58 to 0, Eluent 2: 42 to 100

The column used was Sfar C18, which is manufactured by Biotage. Detection was carried out with an ultraviolet-visible absorption detector (measurement wavelength: 290 nm).

For the liquid obtained through this step, acetonitrile was distilled off under reduced pressure with an evaporator to obtain DOTAGA-DBCO as the purified second compound. The DOTAGA-DBCO is a composition in a state of being dissolved in a 0.1 vol% acetic acid aqueous solution.

Subsequently, the composition was freeze-dried to remove the 0.1 vol% acetic acid aqueous solution, thereby obtaining solid DOTAGA-DBCO. It was stored in a brown vial.

Thereafter, the brown vial containing DOTAGA-DBCO was placed under each of the conditions: room temperature (Example 1: 21 to 25°C); high temperature (Example 2: 40°C); refrigerating (Example 3: 5°C); and freezing (Example 4: -20°C), and stored for a predetermined period.

### [Example 5]

### (Synthesizing Step)

DOTAGA-DBCO was obtained through the method described in the synthesizing step of Examples 1 to 4 except that DMSO was used as an organic solvent to dissolve DOTAGA and DBCO-amine, the molar ratio of the ligand compound to the first compound was 1.2, and the reaction temperature was 80°C.

### (Purifying Step)

The product containing DOTAGA-DBCO was purified in the same manner as in the purifying step of Examples 1 to 4 except that formic acid was used as the water-soluble organic acid for the first and second eluents and elution was performed under the following concentration gradient conditions.

### <Conditions for preparative medium pressure liquid chromatography>

The first eluent (hereinafter, it is also referred to as eluent 1.) and the second eluent (Hereinafter, it is also referred to as eluent 2.) were used for liquid feeding while controlling the concentration gradient (vol%) in the order of the following (i) to (v). The flow rate of each eluent was 12 mL/min.
(i) 0 to 2.8 min Eluent 1: 95, Eluent 2: 5
(ii) 2.8 to 5.7 min Eluent 1: 95 to 80, Eluent 2: 5 to 20
(iii) 5.7 to 19.8 min Eluent 1: 80 to 40, Eluent 2: 20 to 60
(iv) 19.8 to 22.7 min Eluent 1: 40 to 5, Eluent 2: 60 to 95
(v) 22.7 to 25.5 min Eluent 1: 5, Eluent 2: 95

For the liquid obtained through this step, acetonitrile was distilled off under reduced pressure with an evaporator to obtain DOTAGA-DBCO as the purified second compound. The DOTAGA-DBCO is a composition in a state of being dissolved in a 0.1 vol% formic acid aqueous solution.

Subsequently, the composition was freeze-dried to remove the 0.1 vol% formic acid aqueous solution, thereby obtaining solid DOTAGA-DBCO. It was stored in a colorless transparent glass vial.

Thereafter, the colorless transparent glass vial containing DOTAGA-DBCO was placed under the condition of room temperature (Example 5: 19 to 23°C) and stored for a predetermined period.

### [Comparative Examples 1 and 2]

After the synthesizing step was performed in the same manner as in Examples 1 to 4, the product containing DOTAGA-DBCO was subjected to the purifying step in the same manner as in Examples 1 to 4 using an eluent containing TFA. Regarding the eluent in the comparative examples, a 0.1 vol% aqueous TFA solution as the first eluent and a 0.1 vol% TFA-containing acetonitrile as the second eluent were used. Therefore, the composition obtained in the comparative examples contained DOTAGA-DBCO and TFA.

Subsequently, DOTAGA-DBCO obtained by drying in the same manner as in Examples 1 to 4 was put in a brown vial, and the brown vial was placed under each of the conditions: room temperature (Comparative Example 1: 21 to 25°C); and high temperature (Comparative Example 2: 40°C), and stored for a predetermined period.

### [Evaluation of storage stability]

For DOTAGA-DBCOs in each of Examples and Comparative Examples, the purity was measured by the following measurement method at each time point: at the start of storage, after 7 days storage, after 1 month storage, after 3 months storage, and after 6 months storage. The smaller deterioration in purity with respect to the purity at the start of storage, the more excellent the storage stability. The results are shown in Table 1 below.

### <Method for measuring purity>

1.0 mg of the objective compound was precisely weighed. For Examples 1 and 2 and Comparative Examples 1 and 2, a mixture of 10 mmol/L ammonium formate solution (pH 3.0)/acetonitrile for liquid chromatography (95 : 5) was added to make an exactly 10 mL sample solution. For Examples 3 and 4, a mixture of 10 mmol/L ammonium formate solution (pH 3.0)/methanol for liquid chromatography (95 : 5) was added to make an exactly 10 mL sample solution. For Example 5, methanol for liquid chromatography was added to make an exactly 10 mL sample solution.

For 10 µL of each of these sample solutions, high performance liquid chromatography measurement was conducted under the following conditions. The peak area of the sample solution was measured with automatic integration method, and the amount of these compounds was determined through area percentage method. Each sample solution was prepared one time and measured 3 times. The arithmetic average value of the three measurement results was defined as purity (%). Note that, at the start of storage, each sample solution was prepared and measured one time, respectively.

### <Conditions for high performance liquid chromatography>

·Detector (1): ultraviolet absorptiometer (measurement wavelength: 290 nm, 308 nm)
·Detector (2): ACQ-QDa (Positive scan: cone voltage 15 V, capillary voltage 0.8 V; Negative scan: cone voltage 15 V, capillary voltage 0.8 V, scanning range: m/z 50 to 1200)
·Column: a stainless steel tube having an inner diameter of 4.6 mm and a length of 10 cm, packed with 3.5 µm octadecylsilylated silica gel for liquid chromatography (XBridge C18)
·Column temperature: maintained at a constant temperature of about 25°C

### [Examples 1 to 5 and Comparative Examples 1 and 2]

·Mobile phase: (Mobile phase A) 10 mmol/L ammonium formate solution (pH 3.0) and (Mobile phase B) acetonitrile were used for liquid feeding while controlling the concentration gradient (vol%) in the order of the following (i) to (v). The flow rate of each mobile phase was 1 mL/min.
   (i) 0 to 12 min Mobile phase A: 95 to 50, Mobile phase B: 5 to 50
   (ii) 12 to 13 min Mobile phase A: 50 to 5, Mobile phase B: 50 to 95
   (iii) 13 to 20 min Mobile phase A: 5, Mobile phase B: 95
   (iv) 20 to 20.01 min Mobile phase A: 5 to 95, Mobile phase B: 95 to 5
   (v) 20.01 to 30 min Mobile phase A: 95, Mobile phase B: 5

**[Table 1]**

| | Presence or absence of TFA during purification | Compound storage conditions | Purity (%) of compound at each storage time | | | | |
|---|---|---|---|---|---|---|---|
| | | | Storage start | After 7 days | After 1 month | After 3 months | After 6 months |
| Example 1 | Absent | Room temperature | 97.6 | 97.1 | 96.9 | 95.7 | 93.6 |
| Example 2 | Absent | 40°C | 97.6 | 96.9 | 96.7 | 93.9 | 92.0 |
| Example 3 | Absent | 5°C | 96.9 | 97.4 | 97.0 | 93.2 | 96.8 |
| Example 4 | Absent | -20°C | 96.9 | 97.4 | 97.6 | 93.5 | 95.5 |
| Example 5 | Absent | Room temperature | 97.5 | 97.2 | 96.0 | - | - |
| Comparative Example 1 | Present | Room temperature | 96.4 | 95.5 | 90.9 | - | - |
| Comparative Example 2 | Present | 40°C | 96.4 | 86.1 | 52.2 | - | - |

As shown in Table 1, it is found that the compounds of examples, purified in the absence of TFA, can be stored in a state where the purity at the start of storage is maintained even after one month storage, as compared with those of comparative examples. In particular, it is found that Examples 3 and 4, stored under refrigerating or freezing conditions, can be stored in a state where the purity at the start of storage is maintained even after 6 months storage, showing excellent long-term storage stability.

## Claims

1. A manufacturing method for a compound, the manufacturing method comprising:
reacting a ligand compound capable of coordinating to a metal ion with a first compound having an atomic group capable of a click reaction to obtain a product that contains a second compound with a structure having an atomic group capable of a click reaction and a ligand; and subsequently,
subjecting the product to a liquid chromatography method using an eluent that contains a water-soluble organic acid that is liquid under 1 atm at 20 °C and contains no trifluoroacetic acid to obtain a purified second compound.

2. The manufacturing method according to claim 1, wherein the water-soluble organic acid has a boiling point of 150 °C or less under 1 atm.

3. The manufacturing method according to claim 1 or 2, wherein the water-soluble organic acid is acetic acid.

4. The manufacturing method according to any one of claims 1 to 3, wherein the atomic group capable of a click reaction is dibenzocyclooctyne.

5. The manufacturing method according to any one of claims 1 to 4, the method comprising: freeze-drying the purified second compound.

6. The manufacturing method according to any one of claims 1 to 5, wherein the eluent further contains a polar organic solvent.

7. The manufacturing method according to any one of claims 1 to 6, wherein the ligand compound is Macropa, Deferoxamine, HBED-CC, CDTA, DOTA, DTPA, NOTA, or a derivative thereof.

8. The manufacturing method according to any one of claims 1 to 7, wherein the ligand compound and the first compound are reacted with each other through an amidation reaction or a thiourea structure-forming reaction.

9. The manufacturing method according to any one of claims 1 to 7, wherein
an amidation reaction is performed between the ligand compound's carboxy group or oxydicarbonyl group and the first compound's amino group, or
a thiourea structure-forming reaction is performed between the ligand compound's isothiocyanate group and the first compound's amino group.

10. The manufacturing method according to any one of claims 1 to 9, wherein the ligand compound is DOTAGA or an anhydride thereof, and
the first compound is DBCO-amine.

11. A manufacturing method for a targeting agent, the manufacturing method comprising in this order or in the reverse order:
a step of coordinating the second compound obtained by the manufacturing method according to any one of claims 1 to 10 to a metal ion; and
a step of subjecting the second compound and a targeting compound having an atomic group capable of a click reaction to a reaction between the atomic groups capable of a click reaction.

12. The manufacturing method according to claim 11, wherein the metal ion is a radioactive metal ion.

13. A composition comprising:
a compound with a structure having a ligand capable of coordinating to a metal ion and an atomic group capable of a click reaction; and
a water-soluble organic acid that is liquid under 1 atm at 20 °C; wherein
the composition contains no trifluoroacetic acid.

14. The composition according to claim 13, wherein no peaks derived from trifluoroacetic acid are observed when analyzed by ¹⁹F-NMR.

15. The composition according to claim 13 or 14, wherein the atomic group capable of a click reaction is dibenzocyclooctyne.

16. The composition according to any one of claims 13 to 15, wherein the water-soluble organic acid is acetic acid.

17. A compound comprising a structure having a ligand capable of coordinating to a metal ion and an atomic group capable of a click reaction,
wherein, after the compound is stored at -20 °C for 1 month, the compound has a purity decreasing rate of 5% or less relative to a purity that the compound has when the compound starts to be stored.

18. A storing method for a compound, the storing method comprising:
freezing and storing the second compound purified by the manufacturing method according to any one of claims 1 to 10.
